# NEUE EUROPÄISCHE PATENTSCHRIFT

(11) **EP 0 515 768 B2**
(45) Veröffentlichungstag und Bekanntmachung des Hinweises auf die Entscheidung über den Einspruch: **07.01.1998**
(45) Hinweis auf die Patenterteilung: 15.12.1993
(21) Anmeldenummer: 92100090.7
(22) Anmeldetag: 04.01.1992
(51) Int. Cl.: A61K 7/09, A61K 7/06

(54) **Mittel zur Verformung von menschlichen Haaren und Verwendung von Chlorophyll und Chlorophyllderivaten in solchen Mitteln**
Agent for forming human hair and use of chlorophyll and chlorophyll derivatives in such agents
Agent pour la mise en forme des cheveux humains et utilisation de chlorophylle et des dérivés de chlorophylle dans de tels agents

(30) Priorität: 31.05.1991 DE 4117806
(43) Veröffentlichungstag der Anmeldung: 02.12.1992
(73) Patentinhaber: GOLDWELL GmbH, D-64297 Darmstadt (DE)
(72) Erfinder: Rose, Burkhard, W-6100 Darmstadt 13 (DE)

(56) Entgegenhaltungen:
- DE-A- 3 740 926
- JP-A-55 108 812
- PATENT ABSTRACTS OF JAPAN vol. 5, no. 79 (C-56)(751) 23. Mai 1981
- DATABASE WPIL Week 8904, Derwent Publications Ltd., London, GB; AN 89-029128

## Beschreibung

Die vorliegende Erfindung betrifft ein Mittel zur dauerhaften Verformung von menschlichen Haaren, d.h., ein Dauerwellmittel, das eine verbesserte Wellwirkung aufweist.

Bekanntlich erfordert die Dauerwellung zwei Behandlungsschritte:

Die reduktive Spaltung der Cystin-Disulfidbrücken des Haares durch Einwirkung eines Reduktionsmittels und die anschließende Fixierung durch Aufbringung eines Oxidationsmittels, wodurch die Cystin-Disulfidbrücken wieder hergestellt werden.

Das klassische Reduktionsmittel ist, wie bereits aus den Pionierpatenten DE-PS 948 186 und 972 424 hervorgeht, dabei die Thioglykolsäure, z.B. als Ammonium- oder Monoethanolaminsalz, die in den letzten Jahren durch Glycerinmonothioglykolat teilweise ersetzt wurde; jedoch werden auch Thiomilchsäure und deren Ester sowie auch anorganische Sulfite eingesetzt.

Die Thioglykolat enthaltenden Zusammensetzungen weisen dabei einen pH-Wert im Bereich zwischen etwa 7,5 und etwa 9,0, insbesondere 8,5 bis 9,0, auf, wobei die Alkalisierung heute in der Regel, neben Ammoniak, durch Zusatz von Ammonium-(bi)-carbonat erzielt wird (vgl. beispielsweise US-PS 2,708,940).

Die Dauerwell-Zusammensetzungen auf Basis Glycerinmonothioglykolat besitzen einen schwach sauren bis neutralen pH-Wert.

Die Reduktionsmittel-Zusammensetzungen enthalten häufig auch etwa 0,5 bis etwa 35 Gew.-%, vorzugsweise etwa 1, insbesondere etwa 2,5 bis etwa 15 Gew.-% Alkandiole bzw. deren Ether, die auch als Lösungs- und Penetrationsmittel oder Lösungsvermittler bzw. Carrier dienen können.

Diese Zusammensetzungen sind insbesondere hinsichtlich ihrer Wellwirksamkeit jedoch nach wie vor verbesserungsfähig.

Es wurde nun gefunden, daß man die Wellwirkung von Thioverbindungen als Reduktionsmittel enthaltenden Dauerwellmitteln dadurch verbessern und eine Wirksamkeitssteigerung erreichen kann, wenn man ihnen einen geringen Anteil an Chlorophyll bzw. Chlorophyll-Derivaten, insbesondere ein Alkalimagnesium- bzw. kupferchlorophyllin wie Natrium- bzw. Kaliumchlorophyllin, vorzugsweise in einer Menge von 0,01 bis 0,2 Gew.-%, insbesondere 0,02 bis 0,1 Gew.-%, und besonders bevorzugt 0,025 bis 0,075 Gew.-%, berechnet auf die Gesamtzusammensetzung des jeweiligen Mittels, zusetzt.

Unter Dauerwellmitteln im Sinne der Erfindung werden jedoch nicht die Oxidationsmittel enthaltenden Fixierungsmittel verstanden.

Wie bereits ausgeführt, werden als zumindest teilweise wasserlösliche Chlorophyll-Derivate Alkalimagnesiumchlorophylline, d.h. Natrium- und/oder Kaliummagnesiumchlorophylline, eingesetzt. Jedoch können auch Chlorophylline, bei denen das Magnesium ganz oder teilweise durch andere Metalle, insbesondere Kupfer, ersetzt ist, also Alkalikupferchlorophylline, zum Einsatz gelangen.

Als zumindest teilweise wasserlösliches Chlorophyll ist auch ein solches anzusehen, das durch Zusatz von Tensiden wasserlöslich gemacht wurde.

Eine Übersicht über Chlorophylle und Chlorophylline findet sich in dem Review von J.C. Kephart in Econ. Bot. 9(1955), S.3-38, "Chlorophyll Derivatives, Their Chemistry, Commercial Preparation and Uses", auf das hier ausdrücklich Bezug genommen wird.

Die Reduktionsmittel werden in Form von wässrigen Lösungen, Gelen, Emulsionen (Cremes) oder auch als Aerosolschäume eingesetzt und enthalten, neben dem Reduktions- und Alkalisierungsmittel, haarkonditionierende Substanzen, beispielsweise kationische Polymere, gegebenenfalls Verdickungsmittel, sogenannte Carrier, die insbesondere auch die Penetration des Produktes erhöhen, Komplexbildner, Trübungsmittel, Duftstoffe und, zur Verbesserung des Netz- und Penetrationsvermögens, auch oberflächenaktive Substanzen.

Als reduzierende Wirkstoffe in den erfindungsgemäßen Dauerwellmitteln werden, wie bereits erwähnt, neben anorganischen Sulfiten wie Natriumbisulfit, insbesondere Thioglykolsäure und Ammoniumthioglykolat. Thiomilchsäure, deren Salze und Ester, Cystein und dessen Hydrochlorid, Cysteamin, N-Acetylcystein, Thioessigsäure, deren Salze und Estersowie insbesondere Thioglykolsäuremonoglycerinestereingesetzt. Im Falle von dessen Verwendung oder der Verwendung ähnlicher Thioester erfolgt die Vermischung mit der restlichen Reduktionsmittel-Zusammensetzung unmittelbar vor der Applikation.

Die Anwendungskonzentration des reduzierenden Wirkstoffs liegt in Abhängigkeit von der Struktur desselben im allgemeinen zwischen etwa 1 und etwa 15 Gew-% der Reduktionsmittel-Zusammensetzung, vorzugsweise zwischen etwa 3 und etwa 10 Gew.-%.

Die Reduktionsmittel-Zusammensetzung enthält zumeist ein Alkalisierungsmittel.

Die Menge an Alkalisierungsmittel ist abhängig vom reduzierenden Wirkstoff. Vorzugsweise enthält die Reduktionsmittel-Zusammensetzung etwa 1 bis etwa 10, insbesondere etwa 2 bis etwa 8 Gew.-% Alkalisierungsmittel.

Geeignete und bevorzugte Alkalisierungsmittel sind Ammonium(bi)carbonat. Ammoniumcarbamat, Ammoniak und Ethanolamin. Es wird die Einstellung eines pH-Wertes im Bereich zwischen etwa 7 und etwa 8 angestrebt.

Die erfindungsgemäßen Dauerwellmittel enthalten vorzugsweise auch Tenside. Deren Anteil liegt bei 0.1 bis etwa 10, insbesondere etwa 1 bis etwa 5 Gew.-%.

Bei den in den Reduktionsmittel-Zusammensetzungen eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C8-C18-Fettalkoholpolyglykolether, Fettsäurepolyglykolester, Fettsäurealkanolamide, Aminoxide und vor allem C₈-C₁₈-Alkylpolyglykoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer zweckmäßiger Bestandteil der erfindungsgemäßen Zusammensetzungen ist ein C₃-C₆-Alkandiol bzw. dessen Ether, insbesondere Mono-C₁-C₃-alkylether.

Bevorzugte Substanzen sind in diesem Zusammenhang 1,2-und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen etwa 1 und etwa 30, vorzugsweise etwa 2,5 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Propylencaronat (4-Methyl-1,3-dioxolan-2-on), N-Alkylpyrrolidone, Glycerin und Harnstoff Verwendung finden.

Die erfindungsgemäßen Mittel können selbstverständlich alle in Dauerwellmitteln üblichen Stoffe enthalten, auf deren detaillierte Aufzählung hier verzichtet wird.

Zur Vermeidung von Wiederholungen wird vielmehr aufden Stand der Technik verwiesen, wie er beispielsweise in "Ullmann's Encyclopedia of Industrial Chemistry", Vol. A12 (1986), S.588 bis 591, sowie insbesondere der Monographie von K. Schrader, "Grundlagen und Rezepturen der Kosmetika", 2. Aufl. (1989, Hüthig-Verlag), S.823 bis 840 sowie in dem Übersichtsartikel von D.Hollenberg et al in "Seifen-Öle-Fette-Wachse 117 (1981), S.81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Die folgenden Ausführungsbeispiele dienen der näheren Illustration der Erfindung.

### BEISPIEL 1

### Dauerwell-Lösung für normales Haar

- 20,00 (Gew.-%): Ammoniumthioglykolat, 50%ig
- 2,00: Ammoniak, 25%-ig
- 5,00: Ammoniumbicarbonat
- 1,00: C₉-C₁₁-Alkylpolyglykosid (Kondensationsgrad: 1,35)
- 2,50: 1,3-Butylenglykol
- 1,00: Nichtionischer Emulgator (PEG-40-hydriertes Ricinusöl)
- 0,05: Natriumkupferchlorophyllin
- 0,30: Parfum
- ad 100,00: demineralisiertes Wasser

Diese Zusammensetzung wurde im Halbseitenversuch auf die eine Seite des Kopfhaares von 10 Probandinnen aufgebracht, während die andere Seite mit einer identischen Zusammensetzung, jedoch ohne Chlorophyllin behandelt wurde.

Nach 15-minütiger Einwirkungszeit wurde das Haar gespült und mit einer üblichen Fixierlösung der unten angegebenen Zusammensetzung fixiert.

Nach Blindbeurteilung durch zwei Friseure unabhängig voneinander zeigten die mit der in Beispiel 1 beschriebenen Zusammensetzung behandelten Haarhälften eine deutlich verbesserte Wellwirksamkeit gegenüber der anderen Haarhälfte.

### Zusammensetzung der Fixierlösung

- 4,80 (Gew.-%): Wasserstoffperoxid, 50%ig
- 5,00: Natriumlaurylethersulfat, 28%ig
- 0,05: Phenacetin
- 0,50: 1,2-Propandiol
- q.s.: Phosphorsäure zur pH-Einstellung auf 3,0
- ad 100,00: Wasser

### BEISPIEL 2

### Dauerwellmittel für gefärbtes, geschädigtes Haar

- 16,00 (Gew.-%): Ammoniumthioglykolat, 50%ig
- 1,00: Ammoniak, 25%ig
- 2,50: Ammoniumbicarbonat
- 0,50: C₁₀-C₁₂-Alkylpolyglykosid Kondensationsgrad:1,5)
- 0,25: Cetyltrimethylammoniumchlorid
- 0,20: Kationisches Polymer Polyquaternium 10)
- 0,01: Natriummagnesiumchlorophyllin
- 1,00: Nichtionisches Tensid (PEG-40-hydriertes Ricinusöl)
- 0,30: Parfum
- ad 100,00: Enthärtetes Wasser

Diese Zusammensetzung wird mit einem üblichen Fixiermittel auf Basis Wasserstoffperoxid oder Natriumbromat verwendet.

### BEISPIEL 3

### Schaum-Dauerwellmittel für festes, schwer wellbares Naturhaar

- 22,00 (Gew.-%): Ammoniumthioglykolat, 50%ig
- 5,00: Ammoniak, 25%ig
- 4,00: Ammoniumbicarbonat
- 2,50: 1,2-Propandiol
- 1,00: C₁₀-C₁₂-Alkylpolyglykosid (Kondensationsgrad: 1,40)
- 5,00: Kokosamidopropylbetain, 30%ig
- 0,07: Natriumkupfer/magnesiumchlorophyllin
- 1,00: Nichtionischer Emulgator (PEG-40-hydriertes Ricinusöl)
- 0,40: Parfum
- ad 100,00: demineralisiertes Wasser

Die Fixierung erfolgt auf übliche Weise.

### BEISPIEL 4

### Saure Dauerwelle für normales bis leicht geschädigtes Naturhaar

- 25 g 75%iges: Glycerinmonothioglykolat
werden unmittelbar vor der Anwendung mit 75 g einer Lösung aus
- 0.04 g: Natriummagnesium/kupferchlorophyllin
- 1,00 g: Ethylcarbitol
- 0,10 g: Kationisches Polymer Polyquaternium 10)
- 1,00 g: PEG-400-hydriertes Ricinusöl
- 0,50 g: Parfum
- 0,50 g: Laurylpolyglykosid (Kondensationsgrad: 1,75)
- 1,25 g: Ammoniak, 25%ig
- ad 75,00 g: Demineralisiertes Wasser gemischt.

Der pH-Wert der Mischung beträgt ca. 6,8.

Die Fixierung erfolgt mit einer üblichen Oxidationsmittel-Zusammensetzung.

## Patentansprüche

1. Mittel zur dauerhaften Verformung von menschlichen Haaren, enthaltend Thioverbindungen als Reduktionsmittel, dadurch gekennzeichnet, daß es ein zumindest teilweise wasserlösliches Chlorophyll oder Chlorophyll-Derivat enthält.

2. Mittel nach Anspruch 1, dadurch gekennzeichnet, daß es 0,01 bis 0,2 Gew.-% des Chlorophylls oder Chlorophyll-Derivats, berechnet auf die Gesamtzusammensetzung des jeweiligen Mittels, enthält.

3. Mittel nach Anspruch 1 oder 2, dadurch gekennzeichnet, daß es 0,02 bis 0,1 Gew.-%, berechnet auf die Gesamtzusammensetzung des Mittels, des Chlorophylls oder Chlorophyll-Derivats enthält.

4. Mittel nach einem der Ansprüche 1 bis 3, dadurch gekennzeichnet, daß es ein Alkalimagnesiumchlorophyllin und/oder ein Alkalikupferchlorophyllin enthält.

5. Verwendung eines zumindest teilweise wasserlöslichen Chlorophylls oder Chlorophyll-Derivats zur Verstärkung der Wellwirkung in Thioverbindungen als Reduktionsmittel enthaltenden Mitteln zur dauerhaften Verformung von menschlichen Haaren.

6. Verwendung eines Alkalimagnesiumchlorophyllins und/oder Alkalikupferchlorophyllins in Thioverbindungen als Reduktionsmittel enthaltenden Mitteln zur dauerhaften Verformung von menschlichen Haaren.

## Claims

1. Composition for permanent waving of human hair, containing thio compounds as reducing agents, characterized in that it contains a chlorophyll or chlorophyll derivative which is at least partially water-soluble.

2. Composition according to claim 1, characterized in that it contains 0.01 to 2% by weight of the chlorophyll or chlorophyll derivative, calculated to the total reducing composition.

3. Composition according to claim 1 or 2, characterized in that it contains 0.02 to 0.1% by weight of the chlorophyll or chlorophyll derivative, calculated to the total reducing composition.

4. Composition according to one of claims 1 to 3, characterized in that it contains an alkali magnesium chlorophyllin and (or) an alkali copper chlorophyllin.

5. Use of an at least partly water-soluble chlorophyll or chlorophyll derivative to improve the waving performance in compositions for permanent waving of human hair containing thio compounds as reducing agents.

6. Use of an alkali magnesium chlorophyllin and (or) alkali copper chlorophyllin in compositions for permanent waving of human hair containing thio compounds as reducing agents.

## Revendications

1. Produit pour la déformation durable des cheveux humains contenant des composés thio comme les agents réducteurs, caractérisé en ce qu'il contient de la chlorophylle ou un dérivé de chlorophylle au moins partiellement soluble dans l'eau.

2. Produit selon la revendication 1, caractérisé en ce qu'il contient de 0,01 à 0,2% en poids de chlorophylle ou de dérivé de chlorophylle, calculé par rapport à la composition totale du produit.

3. Produit selon la revendication 1 ou 2, caractérisé en ce qu'il contient de 0,02 à 0,1% en poids de chlorophylle ou de dérivé de chlorophylle, calculé par rapport à la composition totale du produit.

4. Produit selon 1 l'une des revendications 1 à 3, caractérisé en ce qu'il contient une chlorophylline de magnésium et de métal alcalin et/ou une chlorophylline de cuivre et de métal alcalin.

5. Utilisation d'une chlorophylle ou d'un dérivé de chlorophylle au moins partiellement soluble dans l'eau, pour le renforcement de l'action d'ondulation de produits pour la déformation durable des cheveux humains contenant des composés thio comme les agents réducteurs.

6. Utilisation d'une chlorophylline de magnésium et de métal alcalin et/ou d'une chlorophylline de cuivre et de métal alcalin dans des produits pour la déformation durable des cheveux humains contenant des composés thio comme les agents réducteurs.
